# EUROPEAN PATENT APPLICATION

(11) **EP 4 570 259 A1**
(43) Date of publication of application: **18.06.2025**
(21) Application number: 23383285.6
(22) Date of filing: 13.12.2023
(51) Int. Cl.: A61K 35/28, A61K 45/06, A61P 1/02

(54) **COMPOSITION FOR USE IN THE TREATMENT OF ORAL DISEASES OR CONDITIONS**

(71) Applicant: Gistem Research S.L., 33204 Gijón Asturias (ES)
(72) Inventor: VIZOSO PIÑEIRO, Francisco Javier, 33204 Gijón (Asturias) (ES); COSTA PÉREZ, Luis Alberto, 33204 Gijón (Asturias) (ES); EIRÓ DÍAZ, Noemí, 33204 Gijón (Asturias) (ES)
(74) Representative: Hoffmann Eitle

(57) **Abstract**

The present invention refers to a composition comprising the secretome of mesenchymal stem cells derived from the uterine cervix, for use in the treatment of oral diseases or conditions.

## Description

### FIELD OF THE INVENTION

The present invention refers to the medical field. Particularly, the present invention refers to a composition comprising the secretome of mesenchymal stem cells derived from the uterine cervix, for use in the treatment of oral diseases or conditions.

### STATE OF THE ART

The dental pulp is a tissue with unique characteristics in the organism, which is part of the dentin-pulp complex located inside the teeth. The rigid structures of enamel and dentine provide mechanical support, defence against the bacterial flora of the mouth and protection from harmful stimuli. The importance of maintaining the vital pulp of a tooth is deduced from the functions it performs throughout its lifespan. Its immune system is able to detect aggressions in the peripheral dentine and trigger protective mechanisms. Its specialised cells called odontoblasts have the capacity to form dentine throughout life. This partly compensates for physiological or pathological loss of enamel and dentine, creating an insulating hard tissue barrier against external irritants. It also contains sensory and autonomic nerves that perform vasomotor and defensive functions.

If the tooth loses its structural integrity either through decay, cracks, fractures or leakage from the margins of a restoration, this creates a pathway for contamination by micro- organisms and toxins from the oral flora.

In the face of bacterial aggression, the dental pulp initially triggers an inflammatory response that will be commensurate with the damage received. Without treatment, it can evolve from reversible inflammation or hyperaemia, then to irreversible pulpitis and end in pulp necrosis. The infection in turn can spread to the periodontal ligament and alveolar bone leading to periapical pathology.

Endodontics, also known as root canal treatment, is the standard therapeutic procedure for permanent teeth with irreversible pulpitis or pulp necrosis. It consists of the complete removal of the affected pulp in two phases: i) chemomechanical preparation and disinfection, ii) obturation with a synthetic or bio- ceramic filling, in order to seal the endodontic space and prevent bacterial recontamination.

When a permanent tooth with a developing root undergoes pulp necrosis, the natural process of root maturation is interrupted. It has been shown that functional forces affect structurally weak, immature teeth to a greater degree, making them prone to fracture and premature loss.

Although the biological basis of endodontics is the treatment of pulpal and periapical pathologies, it has the limitation of not providing the necessary conditions for the revitalisation of the affected tooth.

Research is currently underway to develop an effective treatment to regenerate dental pulp. *In vivo* studies in animal models using dental pulp stem cells (DPSC) have resulted in the generation of tissue with pulp-like characteristics. However, in addition to their high cost, live cell therapies have been shown to have low viability and a survival rate of less than 1% in the host tissue.

In immature teeth with pulp necrosis, clinical treatment alternatives using regenerative endodontic procedures (REP) have been proposed. The aim of REP is the revascularisation of the canal from a blood clot, which acts as an inducer for the migration of nearby stem cells to support new tissue. The technique was described in 1971 with very mixed clinical results. This is probably due to the fact that the knowledge of microbiology and disinfection at that time was insufficient to obtain micro-organism-free canals. Almost 30 years later, new disinfection protocols and working criteria were established, which are still in force today and are included in the "Guide to regenerative endodontic procedures".

An essential factor in endodontic treatment is thorough intra-canal disinfection. Bacterial biofilm must be disorganised through the efficient use of irrigation solutions and topical medications. The anatomical complexity and clinical conditions of infected canals limit the mechanical action of instruments. Irrigation therefore plays a key role in root canal preparation, as it allows disinfection of these inaccessible areas.

In dental revascularisation procedures, both the proper selection of irrigants and their concentration are important. They should have a broad microbicidal spectrum but should not kill dentin collagen and should allow the survival of remaining stem cells. Among the most commonly used substances, sodium hypochlorite (NaOCL) fulfils most of the indispensable requirements as an antibacterial, debridant and instrument lubricant. On the other hand, dentin in its extracellular matrix harbours cytokines and growth factors, which are involved in the proliferation and differentiation of DPSC to odontoblasts. Induction of this effect is achieved by pre-conditioning with 17% ethylene diamine tetraacetic acid (EDTA) solution. Its chelating action releases these biomolecules, and exposes dentin type I collagen, which is necessary for new cell attachment.

An occasional adjunct used in endodontics to complement effective disinfection is topical intra-canal medication. Calcium hydroxide (Ca(OH)₂) is recommended for its antiseptic properties for pulpal necrosis and for its alkalinity also in apexification procedures. As an antibacterial, the use of a combination of 2 or 3 antibiotics is also proposed. However, there are controversies for their use due to side effects such as sensitisation and bacterial resistance, in addition to the fact that they do not cover the entire spectrum of endodontic flora. It was also shown that high concentrations of antibiotics have a detrimental effect on the survival and proliferation of Stem Cell Apical Papilla (SCAP). On the other hand, Ca(OH)₂ placed far from the apex would have a beneficial effect on DPSC or SCAP (Ruparel et al., 2012), but there are reports that prolonged use may increase the likelihood of root fracture.

In the blood clot revascularisation procedure, once the disinfection and conditioning phase of the immature canal has been completed, the periapical tissues are bled until the clot is consolidated at the cervical level of the tooth.

A biomaterial, Mineral Trioxide Aggregate (MTA), is then placed. The sealing of the dental crown is performed by filling with composite resin bonded to the dental tissues.

Several *in vivo* studies have shown that the application of REP can promote the neoformation of blood vessels and tissues inside the canals. However, in most cases the neoformed tissue is not identical to the original dental pulp, but rather similar to root cementum, bone or fibrous connective tissue with periodontal characteristics. It follows, therefore, that despite the advances made, pulp regeneration currently lacks an alternative treatment with predictable results.

Research based on tissue engineering has attempted the neoformation of dental pulp through biomaterials, stem cells and growth factors. However, problems of dosing and release of these factors, coupled with safety concerns regarding the use of living cells, limit the translational potential of these approaches.

So, there is an unmet medical needed of finding alternative and effective treatments of oral diseases or conditions. The present invention is focused on solving this problem and the secretome, also known as conditioned medium (CM), derived from human uterine cervical stem cells (CM-hUCESC) and endovesicles (EV) derived from human uterine cervical stem cells (EV-hUCESC) are herein proposed as an effective treatment of oral diseases or conditions.

### DESCRIPTION OF THE INVENTION

### Brief description of the invention

The present invention refers to a composition comprising the secretome of mesenchymal stem cells derived from the uterine cervix, for use in the treatment of oral diseases or conditions.

Such as it can be seen in **Example 2,** the secretome of mesenchymal stem cells derived from the uterine cervix (CM-hUCESC) significantly promotes the proliferation of dental pulp stem cells (DPSC) (**Example 2.1**). On the other hand, *in vivo* studies in mice, the treatment with secretome of mesenchymal stem cells derived from the uterine cervix regenerated tissue within the dental root, with characteristics similar to connective tissue, showing vascular and nerve tissue (**Example 2.2**). Moreover, in *in vivo* studies in dogs, the treatment with secretome of mesenchymal stem cells derived from the uterine cervix induced the formation of tissue inside the canal, the formation of the dentine bridge as well as the characteristic palisade cell structure of the odontoblasts, vessels and nervous tissue (**Example 2.3**). Please note that these results were shown with conditioned medium and endovesicles, both forming part of the secretome.

Consequently, the present invention offers clear evidences that the secretome of mesenchymal stem cells derived from the uterine cervix may be used for the treatment of oral diseases or conditions.

So, the first embodiment of the present invention refers to a composition comprising the secretome of mesenchymal stem cells derived from the uterine cervix, for use in the treatment of oral diseases or conditions.

In a preferred embodiment, the present invention refers to a composition comprising the secretome of mesenchymal stem cells derived from the uterine cervix for use in the treatment of periodontal, gingival and/or tooth diseases.

In a preferred embodiment, the present invention refers to a composition comprising the secretome of mesenchymal stem cells derived from the uterine cervix for use in the regeneration of tooth tissues comprising: Enamel, dentin, and/or pulp tissue and/or periodontal tissue comprising: Gingiva, cementum, alveolar bone, the periodontal ligament and/or oral mucosa.

In a preferred embodiment, the secretome is contained in a cell culture liquid obtained by culturing mesenchymal stem cells derived from the uterine cervix in a cell culture medium, and wherein the secretome is obtained by removing the cells from the culture medium.

In a preferred embodiment, the secretome of mesenchymal stem cells derived from the uterine cervix is dissolved in a scaffold.

In a preferred embodiment, the secretome of mesenchymal stem cells derived from the uterine cervix is dissolved in a hydrogel scaffold as provided for instance by [Garcia-Del Rio, Lorena et al. "New tools to design smart thermosensitive hydrogels for protein rectal delivery in IBD. " Materials science & engineering. C, Materials for biological applications vol. 106 (2020): 110252. doi:10.1016/j.msec.2019.110252]*.*

In a preferred embodiment, the secretome of mesenchymal stem cells derived from the uterine cervix is administered dissolved in a hydrogel scaffold inside the root canal up to the apex, preferably using a pre-filled syringe and needle, in-situ as a constituent part of a periodontal graft or as a gingival dressing.

The second embodiment of the present invention refers to a composition comprising the secretome of mesenchymal stem cells derived from the uterine cervix dissolved in a scaffold.

In a preferred embodiment, the composition comprising the secretome of mesenchymal stem cells derived from the uterine cervix is dissolved in a hydrogel scaffold, forms part of the periodontal graft or of the gingival dressing.

In a preferred embodiment, the secretome comprises the following growth factors and/or cytokines with angiogenic activity: VEGF, Angiogenin, PIGF and/or HGF.

In a preferred embodiment, the secretome comprises the following growth factors and/or cytokines with osteoinductive activity: BMPs, TGF-β, and/or Osteoprotegerin.

In a preferred embodiment, the secretome comprises the following growth factors and/or cytokines with neurotrophic activity: CNTF, NT-3, GDNF, SCF and/or BDNF.

On the other hand, the present invention also refers to a method for treating oral diseases or conditions, preferably for treating periodontal, gingival and/or tooth diseases, more preferably for regenerating tooth tissues comprising: enamel, dentin, and/or pulp tissue and/or periodontal tissue comprising: gingiva, cementum, alveolar bone, the periodontal ligament and/or oral mucosa, wherein the method comprises administering a therapeutically effective dose or amount of the secretome of mesenchymal stem cells derived from the uterine cervix.

For the purpose and interpretation of the scope of the present invention the following terms are defined:
- "Secretome", (also referred to as the stromal cell secretome or conditioned medium), is a collective term for the paracrine soluble factors produced by stem cells and utilized for their inter-cell communication. In addition to inter-cell communication, the paracrine factors are also responsible for tissue development, homeostasis and regeneration. The stem cell secretome comprises extracellular vesicles, specifically exosomes, microvesicles, membrane particles, peptides and small proteins (cytokines).
- "Oral disease or condition" encompass a range of diseases and conditions comprising periodontal, gingival, tooth diseases or diseases affecting the oral mucosa.
- "Periodontal disease" refers to a set of inflammatory conditions affecting the tissues surrounding the teeth. In its early stage, called gingivitis, the gums become swollen and red and may bleed. In its more serious form, called periodontitis, the gums can pull away from the tooth, bone can be lost, and the teeth may loosen or fall out. The tissues surrounding the teeth which are involved in the periodontal disease (periodontium) are the specialized tissues that both surround and support the teeth, maintaining them in the maxillary and mandibular bones. The word comes from the Greek terms περí peri-, meaning "around" and -odont, meaning "tooth". Literally taken, it means that which is "around the tooth". It consists of four principal components, namely: Gingiva, Periodontal ligament (PDL), Cementum and Alveolar bone proper.
- "Gingival disease" is the term given to any disorder primarily affecting the gingiva. An example is gingivitis. The gums or gingiva consist of the mucosal tissue that lies over the mandible and maxilla inside the mouth.
- "Tooth disease" is any condition or disease affecting the teeth that can be congenital or acquired. So, it is any condition or disease affecting the enamel, the dentin and/or the pulp.
- "Scaffolds" refers to any material which provides bulk and mechanical structures to the composition of the invention. The composition of the invention may be suspended, dissolved or adhered to the scaffold framework. The scaffold may be a hydrogel scaffold or any other material which provides bulk and mechanical structures to the composition of the invention, such as a graft, patch or dressing.
- The term "comprising" means including, but it is not limited to, whatever follows the word "comprising". Thus, use of the term "comprising" indicates that the listed elements are required or mandatory, but that other elements are optional and may or may not be present.
- By "consisting of" means including, and it is limited to, whatever follows the phrase "consisting of'. Thus, the phrase "consisting of" indicates that the listed elements are required or mandatory, and that no other elements may be present.
- By "therapeutically effective dose or amount" of the composition of the invention comprising is intended an amount that, when administered as described herein, brings about a positive therapeutic response in a subject having an oral disease or condition. The exact amount required will vary from subject to subject, depending on the species, age, and general condition of the subject, the severity of the condition being treated, mode of administration, and the like. An appropriate "effective" amount in any individual case may be determined by one of ordinary skill in the art using routine experimentation, based upon the information provided herein.

### Description of the figures

**Figure 1****.** Graph of percentage values of proliferation of DPSC cultured in culture medium (DMEM), conditioned medium obtained from DPSC (CM-DPSC) and conditioned medium obtained from hUCESC (CM-hUCESC). Data expressed as mean ± standard deviation (*) p<0.05 vs. CM-DPSC.
**Figure 2****.** Photographic record at day 5 of culture of DPSC in the control group **(A and B)** and with CM-hUCESC **(C and D)** in the presence of dentine fragments (d). In the control group **(B)** there is little cell proliferation, while in those treated with CM-hUCESC **(D)** there are several layers of cells in contact with the dentine fragment and oriented towards it. Objective X40.
**Figure 3****.** Sequence of the 3 stages of photographic recording of cultured DPSC in the presence of dentine fragments (d). The control (untreated) group from **(A)** to **(C)** and the CM-hUCESC treated cells from **(D)** to **(F).**
**Figure 4****.** Analysis of gene expression levels for DMP1 **(A),** DSPP **(B)** and OCN **(C).** Data expressed as mean ± standard deviation (*) p<0.05.
**Figure 5****.** Results of the DPSC gene expression profile of factors with neurotrophic activity. It is observed that CM-hUCESC significantly stimulates the expression of key factors for nerve tissue regeneration such as BDNF and NT3. Data expressed as mean ± standard deviation (*) p<0.05.
**Figure 6****.** The gene expression of VEGF, with a known angiogenic activity, by DPSC is significantly increased in cells treated with CM-hUCESC. Data expressed as mean ± standard deviation (*) p<0.05.
**Figure 7****.** Growth factors and most relevant cytokines of CM-hUCESC with angiogenic activity, compared with those of CM-ASC (adipose tissue-derived mesenchymal stem cells) and control medium. Data expressed as the mean.
**Figure 8****.** Growth factors and Cytokines of known osteoinductive activity identified in the CM-hUCESC, compared to those in the CM-ASC (adipose tissue-derived mesenchymal stem cells) and control medium. Data expressed as the mean.
**Figure 9****.** Growth factors and cytokines with neurotrophic activity in the process of nerve tissue formation. Data expressed as the mean.
**Figure 10****.** Sample at X100 magnification of a root of the control group filled with HG processed by the cut and polish system, in which no tissue is visible inside.
**Figure 11****.** Sample at X100 magnification processed by decalcification of a root canal from the control group filled with HG, showing some remains of hydrogel without tissue inside. Staining with H&E.
**Figure 12****.** Representative images of the regenerated tissue inside the root canal of the experimental group with CM-hUCESC/HG processed by cutting and polishing. Magnification X100 **(A)** and X200 **(B)** and **(C).**
**Figure 13****.** Representative images of neoformed tissue from two roots of the experimental group with CM-hUCESC/HG processed by decalcification. Staining with H&E. Magnification X100 **(A)** and X200 **(B).**
**Figure 14****.** Representative images of cellular structures in roots of the experimental group with CM-hUCESC/HG. Histological technique of cut-polish and H&E staining. Magnification X400.
**Figure 15****.** Immunohistochemical staining of the neoformed tissue inside the tooth roots of the experimental group with CM-hUCESC/HG. The blue arrows indicate the new blood vessels. Magnification: **(A)** X200; **(B)** and **(C)** X400.
**Figure 16****.** Representative images of neoformed tissue within the dental root, with immunohistochemical staining of three roots from the experimental group with CM-hUCESC/HG. Arrows indicate new neural structures identified through S-100 protein expression. Magnification: **(A)** X200; **(B)** and **(C)** X400.
**Figure 17****.** Representative images of neoformed tissue within the canine dental root. Arrows indicate the relevant structure observed. CM-hUCESC/HG; EVs-hUCESC/HG; EVs-hUCESC/HG X400.
**Figure 18****.** Representative images of immunostaining of **(A)** DSPP and **(B)** S100 of CM-hUCESC/HG treated canine root.

### Detailed description of the invention

The present invention is illustrated by means of the Examples set below without the intention of limiting its scope of protection.

### Example 1. Material and Methods

### Example 1.1. Mesenchymal stem cell isolation and characterization

Uterine cervical mesenchymal stem cells were obtained from hysterectomy specimens in patients with no infectious, immunological or tumour pathology. The cells were isolated by mechanical disaggregation followed by enzymatic digestion. Subsequently, the cells were centrifuged 5 min at 400g, and the cell button was resuspended in DMEM-F12 culture medium supplemented with glutamine, penicillin, streptomycin, 10% fetal bovine serum (FBS), epidermal growth factor, hydrocortisone and insulin. They were seeded and incubated at 37°C, air-carbon dioxide (CO₂) atmosphere (95:5) and 90% humidity.

Characterisation of mesenchymal stem cells from uterine cervix (hUCESC) was performed using antibodies specific for the following surface markers: CD31, CD34, CD44, CD45, CD73, CD90, CD105, CD117 according to the International Society for Cell Therapy Committee on Mesenchymal and Tissue Stem Cells (Dominici et al., 2006).

### Example 1.2. Secretome production

Cells were cultured in DMEM-F12 culture medium with 10% FBS, streptomycin 0.1 mg/ml and 100 IU/ml penicillin, at 37 °C, in air-CO₂ atmosphere (95:5), until medium-high confluence was reached. After washing, the cells were again cultured with DMEM-F12 without additives for 48 hours, in an incubator at 37°C, in an air-CO₂ (95:5) atmosphere and 90% humidity.

### Example 1.3. In vitro studies

### Example 1.3.1. Effect of secretome on dental pulp stem cells

### Example 1.3.1.1. Proliferation

To assess the effect of hUCESC secretome/conditioned medium on DPSC proliferation, conditioned DPSC medium (CM-DPSC) was obtained in a manner analogous to that performed for CM-hUCESC production. DPSC were seeded and cultured for 48 hours at 37 °C and 5% CO2 to allow cell adhesion. Subsequently, DPSC were treated with DMEM-F12 (control), CM-DPSC or CM-hUCESC for 48h.

Proliferation was quantified by measuring mitochondrial activity using WST-1 reagent and a plate reader (BioRad, iMarkTM).

### Example 1.3.1.2. Proliferation and dentine fragments

DPSC were seeded on plates with DMEM-F12 with 5% FBS and antibiotics. After 5 days of culture, dentine fragments were seeded, and cells were treated with DMEM-F12 (control) or CM-hUCESC. Media were renewed every 48h-72h. A photographic record was taken on days 1, 5 and 7 of the experiment. On day 7, cells were collected for molecular study.

RNA was extracted from DPSC and the expression of DMP-1, DSPP, OCN, VEFGA, BDNF and NTF-3 genes was identified by qPCR.

### Example 1.3.2. CM-hUCESC growth factors and cytokines

The conditioned medium of lyophilised hUCESC was profiled using RayBio^{®} Human Cytokine Antibody Array G-Series 2000 (Norcross, USA), which simultaneously detects 174 human cytokines. DMEM-F12 culture medium without FBS was processed in parallel as a negative control. For comparative analysis, cytokines from conditioned adipose-tissue stem cell-conditioned medium (CM-ASC) were identified with identical procedure.

Experimental steps and analyses were performed according to the manufacturer's instructions. Signal intensity values representing the detected cytokines were subtracted from the background and normalised to positive controls on the same membrane.

### Example 1.4. In vivo studies

### Example 1.4.1. Studies in mice

The species Mus musculus, strain balb nu/nu, female, 7 weeks old, has been used for this trial. A total of 12 human uniradicular teeth extracted for orthodontic reasons or periodontal disease were used. The characteristics regarding donor ages and tooth manipulation were identical to those described above. A complete root model was developed for this study, thus modifying previous experimental designs (Huang et al., 2010; Li et al., 2016) in order to mimic the real situation clinically encountered in immature permanent teeth. Tooth roots were standardised to a length of 12 mm by making two cuts, one at 3 mm from the apex and the other at the cementum-enamel junction. The remaining pulp tissue was removed, and the root canals were prepared for product injection. One group of roots were filled with CM- hUCESC dissolved in hydrogel through the apical foramen, using a pre-filled syringe and needle, and the control group of roots were filled with the hydrogel.

With the animal anaesthetised, incisions were made on the right and left sides of the lower dorsum and gentle dissection of the subcutaneous plane generating subdermal pockets (Cooper et al., 2000; Huang et al., 2010; Messier et al., 1999; Prescott et al., 2008). One root from the control group (hydrogel) was inserted on the left side and one root from the experimental group (CM-hUCESC/hydrogel) on the right. The incisions were sutured with PGA 4.0 (SSA90. Lorca Marin. Murcia. Spain) and in some cases staples were used to ensure wound synthesis.

Eight weeks after implantation, post-mortem surgical recovery of the dental specimens was performed. The tooth roots of the experimental and control groups were fixed in 10% PFA for 24h for further histological processing. On the one hand, 8 tooth roots were randomly selected, 4 from the experimental group and 4 controls were decalcified and embedded in paraffin for immunohistochemical staining.

On the other hand, 4 remaining samples (2 experimental and 2 controls) were processed by cut-polish technique and haematoxylin-eosin staining was performed for the identification of the different structural elements of relevance.

### Example 1.4.2. Studies in dog

For the studies in a canid animal model, 6 male beagle dogs of approximately 5 months of age were used, with the roots of the premolars in the development phase (Thibodeau et al., 2007) (Claus et al., 2004), as at this stage they conserve the apical papilla as a reservoir of SCAP with odontogenic capacity (Estrela et al., 2011). The animals are microchipped by the supplier in accordance with current legislation. Six premolars of each animal were treated after anastasis of the animals. The inside of the canals of the 2nd, 3rd and 4th lower premolars and the 2nd and 3rd upper premolars were endodontically prepared and filled according to the assigned treatment: CM-hUCESC/HG (hydrogel), or extracellular vesicles derived from CM-hUCESC in hydrogel (EV-hUCESC/HG), using only HG in one premolar as a negative control and leaving one premolar intact as a control of the natural evolution of the teeth in their root development.

A total of 120 roots of 60 birradicular premolars were studied for the experiment, taking each root individually as the unit of measurement.

### Example 2. Results

### Example 2.1. Effect of secretome on dental pulp stem cells

### Example 2.1.1. Proliferation of DPSC

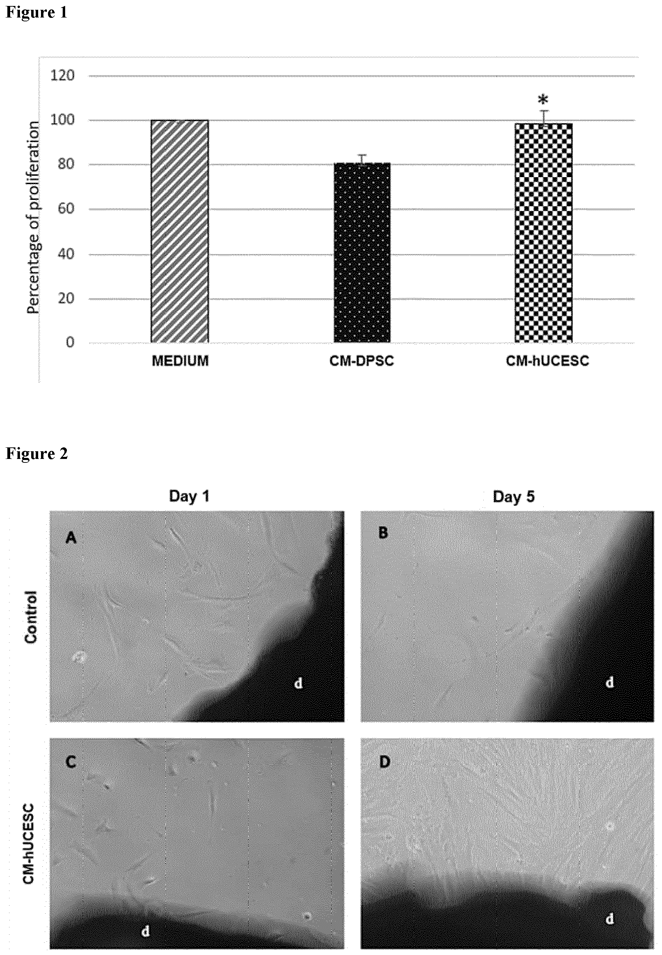

The results of the study show that CM-hUCESC significantly promotes the proliferation of DPSC, relative to CM-DPSC (**Figure 1**). In the proliferation study, an increase in DPSC proliferation was observed in the group treated with CM-hUCESC compared to CM-DPSC.

### Example 2.1.1.1. Proliferation of DPSC cultured with dentine fragments

During the established culture time, DPSC were photographed to assess their morphology and proliferative activity. After the first few days of culture, homogeneous populations of cells were observed whose morphology and behaviour were consistent with those described in previous work for DPSC (Gronthos et al., 2000; Romero et al., 2014). The cells had a spindle-shaped appearance, with short cytoplasmic extensions and a bulky and centred nucleus. On day 5 of culture, DPSC seeded with tooth fragments showed unequal proliferative activity between the groups, being more active in the group of cells cultured in the presence of CM-hUCESC (**Figure 2**).

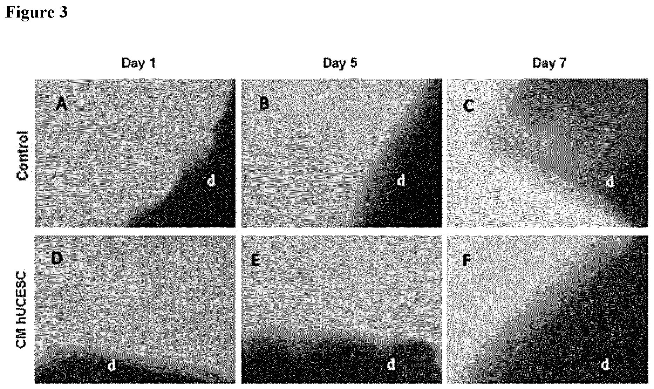

After 7 days of culture, DPSC from both groups were observed to be in contact with the surface of the dentine fragment, being comparatively higher in those treated with CM-hUCESC, being organised in superimposed layers of cells oriented towards or directly in contact with the dentine fragment (**Figure 3**).

The CM-hUCESC-treated cells show increased proliferative activity from day 5 (E) and being evident at day 7 (F) where DPSC are present in numerous clusters attached to dentin and in overlapping layers.

In the control group at day 7 (C) a certain increase of cells is observed with respect to day 5 (B), also oriented towards or in contact with the dentine fragment. Objective X40.

### Example 2.1.1.2. Gene expression of DMP-1, DSPP, OCN, VEFGA, BDNF and NTF-3 by DPSC

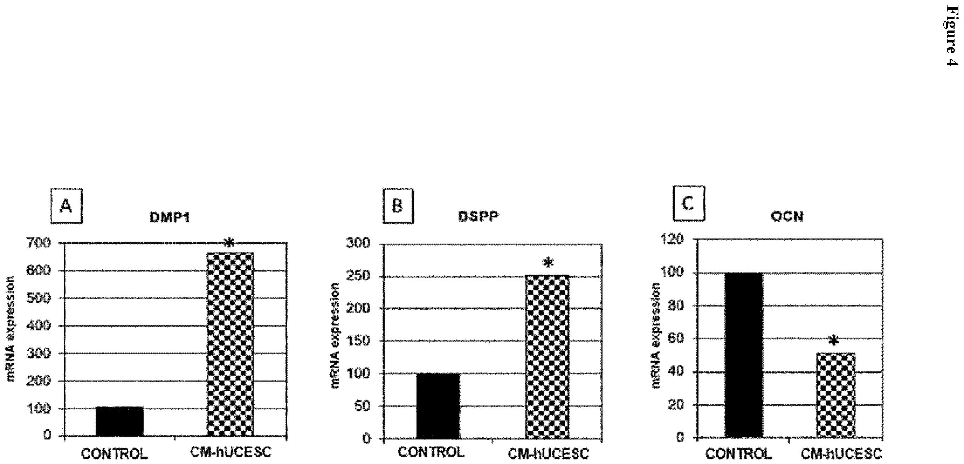

The results obtained by RT-PCR show a significant increase in the expression of the DMP1 gene in the group of DPSC treated with CM-hUCESC, which demonstrates an early stage of differentiation towards odontoblasts (**Figure 4A**). High levels of DSPP gene expression were recorded for CM-hUCESC-treated DPSC, with values more than two times higher than the control, corroborating their differentiation into odontoblasts (**Figure 4B**). However, the highest OCN expression is observed in the control group (untreated DPSC), indicating the presence of DPSC in the late stage of differentiation towards odontoblasts or osteoblasts, compared to the CM-hUCESC-treated group where OCN expression levels are approximately half (**Figure 4C**).

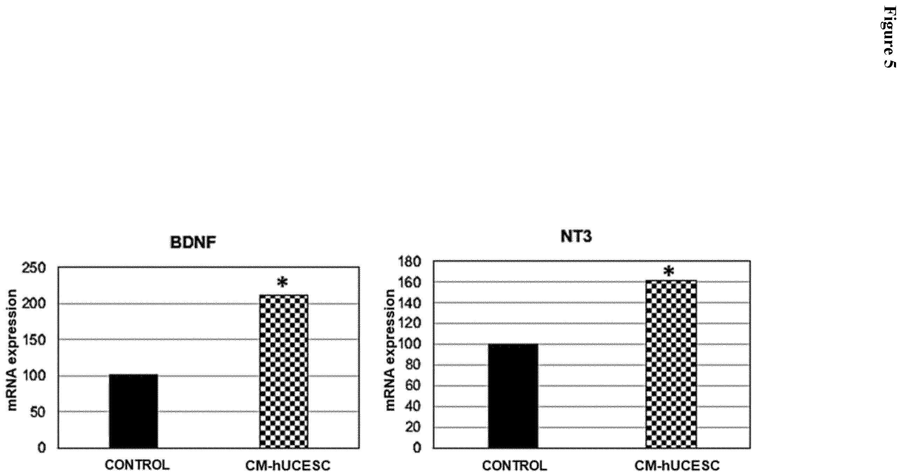

In relation to the analysis of the expression of factors with neurotrophic activity by DPSC, the results show that cells treated with CM-hUCESC produce significantly elevated values of NT-3 and, in addition, the expression of BDNF is more than double that of the control (**Figure 5**).

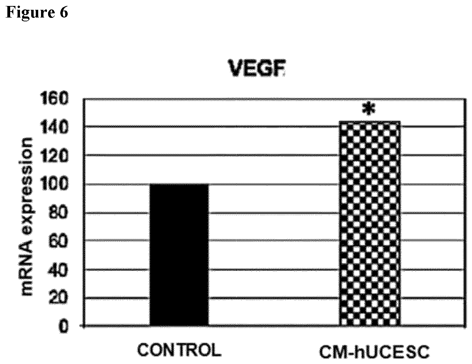

The significantly high expression of the vascular endothelial growth factor VEGF in CM-hUCESC-treated DPSC demonstrates that this biological derivative creates the necessary conditions to favour angiogenesis (**Figure 6**).

### Example 2.1.2. CM-hUCESC growth factors and cytokines

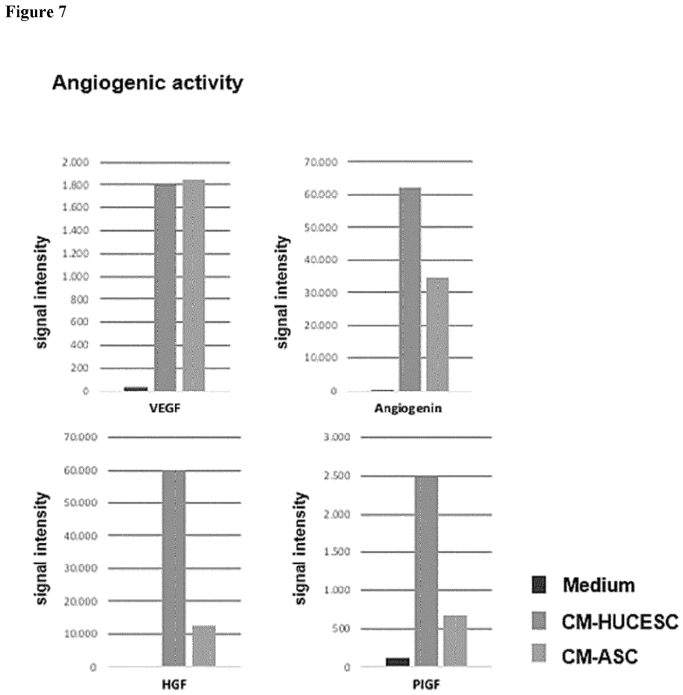

**Figures 7****,** **8** **and** **9** represent the plots of growth factors (GF) and cytokines with marked angiogenic, osteogenic and neurotrophic activity, respectively. The signal intensity values of each element are presented in the graphs as the arithmetic mean in CM-hUCESC, CM-ASC and control medium.

**Figure 7** shows that the CM-hUCESC expresses high values of VEGF, doubling the expression of Angiogenin, and even 5 times more for PIGF and HGF in relation to the CM-ASC.

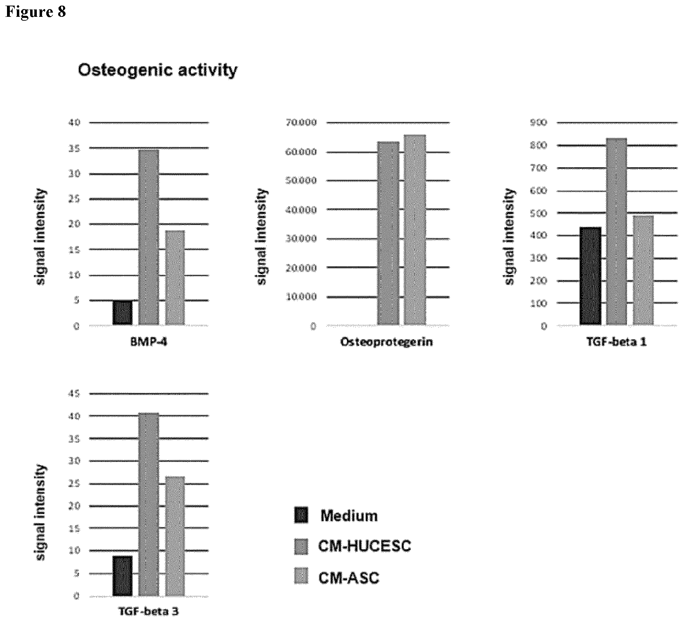

In **Figure 8****,** high expression of BMP-4, TGF-β1, TGF-β3 is observed in CM-HUCEC, practically doubling the values of CM-ASC and with similar results of Osteoprotegerin in both conditioned media.

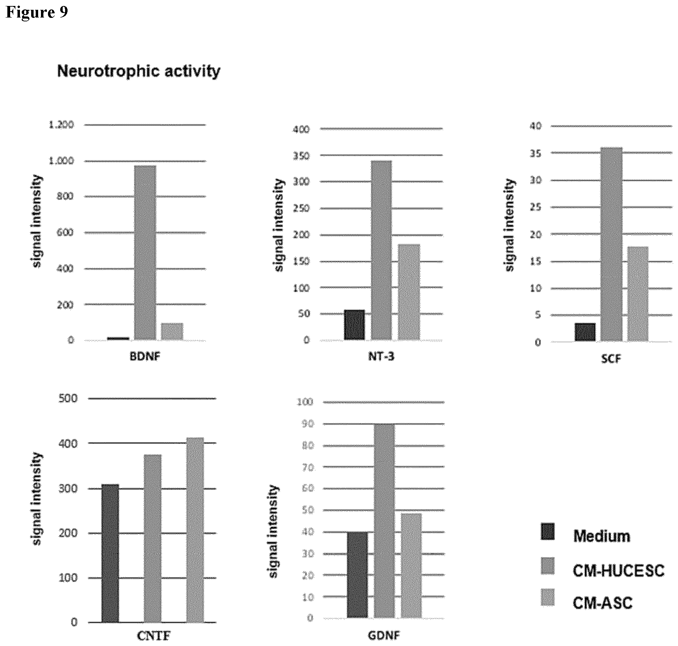

In relation to molecules with neurotrophic activity, it is observed that the CM-hUCESC expresses NT-3, GDNF and SCF in values that double the CM-ASC and almost eight times more in the case of BDNF **(****Figure 9****).**

### Example 2.2. In vivo studies in mice

### Example 2.2.1. Histology

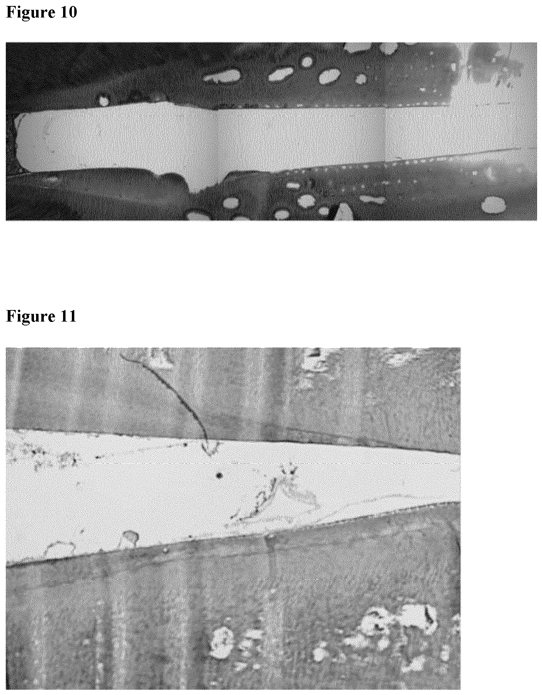

Each human root was examined histologically as an independent sample unit, using a semiautomatic light microscope (Olympus BX61) with built-in camera (Olympus DP72). Inside all canals of the hydrogel control group, no tissue regeneration of any kind was observed (**Figures 10 and 11**).

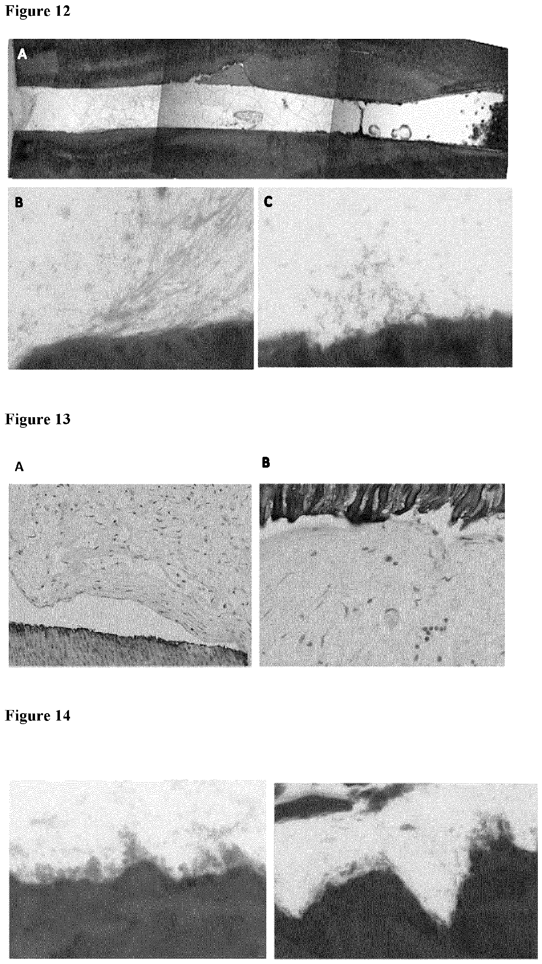

In the roots of the experimental group with CM-hUCESC/HG (hydrogel) inside, regenerated tissue with characteristics similar to connective tissue is observed, highlighting the presence of blood vessels and groups of cells organised in the form of a "palisade" in contact with the dentine walls (**Figure 12** and **Figure 13**).

The neoformed tissues inside the canals show some of the characteristics similar to dental pulp. **Figure 12A** shows the presence of the new tissue, which in all samples occupied two thirds of the total length. In **Figure 12B**, solid structures of the new tissue are observed anchored in the apical access zone of the root. In **Figure 12C**, cells of the new tissue can be seen arranged in layers on the dentine surface.

As can be seen in **Figure 13**, the structural characteristics of the neoformed tissue are similar to those of loose connective tissue, with a predominance of collagen and fibroblasts. The gaps observed at the tissue-dentine interface **Figure 13A** are artefacts probably caused during histological processing by decalcification. These unwanted effects have prevented the identification of cellular structures on the canal surface **Figure 13A** and **Figure 13B**, clearly observed in figure 37 of hard tissue microtome sections.

In hard tissue microtome sections and H&E staining, cell clusters were observed in a "cell palisade" arrangement in direct contact with the root canal dentine (**Figure 14**). This feature resembles the organisation of odontoblasts in the dental pulp.

### Example 2.2.1. Immunohistochemistry

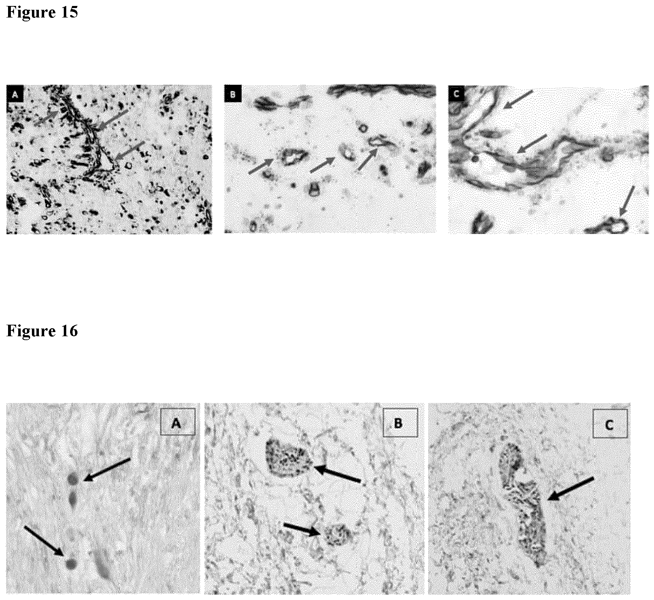

By immunohistochemical staining of the neoformed tissue in the experimental group's human tooth roots filled with CM-hUCESC/HG, abundant vascular structures were identified (**Figure 15**)**.** In **(A)** the presence of new blood vessels can be seen. In images **(B)** and **(C)** the blood vessels of the new tissue at higher magnification, identified as CD-31 positive vascular endothelium.

The presence of nerve tissue was identified through the expression of S-100 protein in CM-hUCESC/HG treated roots (**Figure 16**). New cells **(A)** and well-organised neural structures forming bundles of nerve fibres **(B** and **C)** are observed.

### Example 2.3. In vivo studies in dogs

### Example 2.3.1. Histology

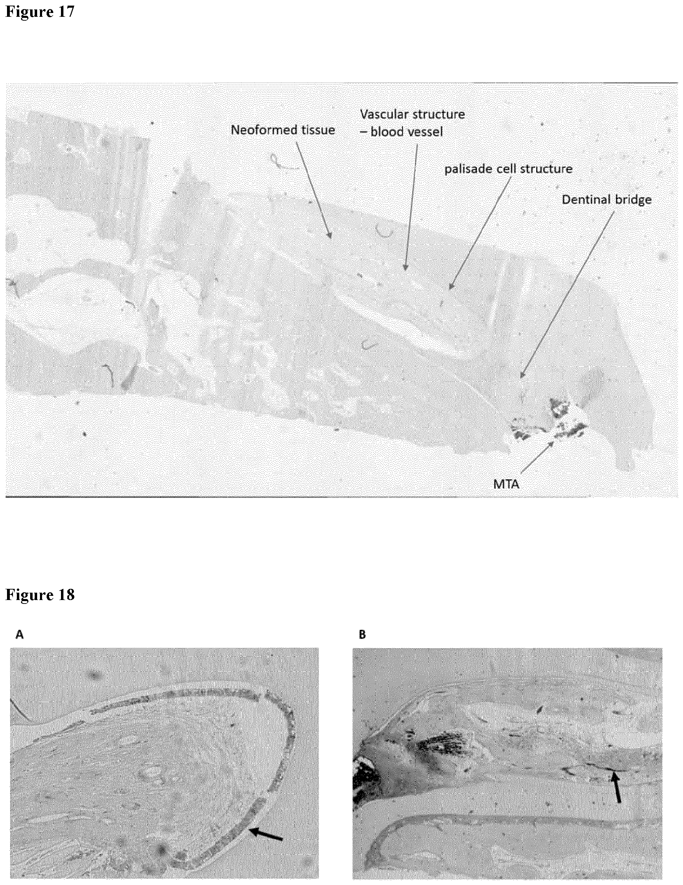

The histology of roots treated with hydrogel or CM-hUCESC/HG has been evaluated, showing that filling the root canal with CM-hUCESC/HG induces the formation of tissue inside the canal, the formation of the dentinal bridge, and blood vessels (angiogenesis), as well as the characteristic palisade cell structure of the odontoblasts (**Figure 17**). No significant differences have been evidenced between CM-hUCESC/HG and EVs-hUCESC/HG.

### Example 2.3.1. Immunohistochemistry

The expression of DSPP protein was observed in the neoformed tissue, which is evidence that CM-hUCESC/HG and EVs-hUCESC/HG induce tissue regeneration including odontoblasts. Also, the evaluation of expression of S100 protein evidenced the presence of nervous tissue in the teeth treated with CM-hUCESC/HG and EVs-hUCESC/HG, similar to the control teeth (intact teeth), but not in the teeth treated with HG alone (**Figure 18**).

## Claims

1. A composition comprising the secretome of mesenchymal stem cells derived from the uterine cervix, for use in the treatment of oral diseases or conditions.

2. Composition comprising the secretome of mesenchymal stem cells derived from the uterine cervix for use, according to claim 1, in the treatment of periodontal, gingival and/or tooth diseases.

3. Composition comprising the secretome of mesenchymal stem cells derived from the uterine cervix for use, according to any of the previous claims, in the regeneration of tooth tissues comprising: Enamel, dentin, and/or pulp tissue and/or periodontal tissue comprising: Gingiva, cementum, alveolar bone, the periodontal ligament and/or oral mucosa.

4. Composition comprising the secretome of mesenchymal stem cells derived from the uterine cervix for use, according to any of the previous claims, wherein the secretome is contained in a cell culture liquid obtained by culturing mesenchymal stem cells derived from the uterine cervix in a cell culture medium, and wherein the secretome is obtained by removing the cells from the culture medium.

5. Composition comprising the secretome of mesenchymal stem cells derived from the uterine cervix for use, according to any of the previous claims, wherein the secretome comprises the following growth factors and/or cytokines with angiogenic activity: VEGF, Angiogenin, PIGF and/or HGF.

6. Composition comprising the secretome of mesenchymal stem cells derived from the uterine cervix for use, according to any of the previous claims, wherein the secretome comprises the following growth factors and/or cytokines with osteoinductive activity: BMPs, TGF-β, and/or Osteoprotegerin.

7. Composition comprising the secretome of mesenchymal stem cells derived from the uterine cervix for use, according to any of the previous claims, wherein the secretome comprises the following growth factors and/or cytokines with neurotrophic activity: CNTF, NT-3, GDNF, SCF and/or BDNF.

8. Composition comprising the secretome of mesenchymal stem cells derived from the uterine cervix for use, according to any of the previous claims, wherein the secretome is dissolved in a scaffold.

9. Composition comprising the secretome of mesenchymal stem cells derived from the uterine cervix for use, according to any of the previous claims, wherein the secretome is dissolved in a hydrogel scaffold.

10. Composition comprising the secretome of mesenchymal stem cells derived from the uterine cervix for use, according to any of the previous claims, wherein the method comprises administering the secretome dissolved in a hydrogel scaffold inside the root canal up to the apex, preferably using a pre-filled syringe and needle, in-situ as a constituent part of a periodontal graft or as a gingival dressing.

11. Composition comprising the secretome of mesenchymal stem cells derived from the uterine cervix dissolved in a scaffold.

12. Composition comprising the secretome of mesenchymal stem cells derived from the uterine cervix, according to claim 11, dissolved in a hydrogel scaffold, form part of the periodontal graft or of the gingival dressing.

13. Composition comprising the secretome of mesenchymal stem cells derived from the uterine cervix dissolved in a scaffold, according to any of the claims 11 or 12, wherein the secretome comprises the following growth factors and/or cytokines with angiogenic activity: VEGF, Angiogenin, PIGF and/or HGF.

14. Composition comprising the secretome of mesenchymal stem cells derived from the uterine cervix dissolved in a scaffold, according to any of the claims 11 to 13, wherein the secretome comprises the following growth factors and/or cytokines with osteoinductive activity: BMPs, TGF-β, and/or Osteoprotegerin.

15. Composition comprising the secretome of mesenchymal stem cells derived from the uterine cervix dissolved in a scaffold, according to any of the claims 11 to 14, wherein the secretome comprises the following growth factors and/or cytokines with neurotrophic activity: NT-3, GDNF, SCF and/or BDNF.
